# FASCICULE DE BREVET EUROPEEN

(11) **EP 3 160 520 B1**
(45) Date de publication et mention de la délivrance du brevet: **28.02.2024**
(21) Numéro de dépôt: 15753082.5
(22) Date de dépôt: 25.06.2015
(51) Int. Cl.: A61L 2/238

(54) **UTILISATION DE MATÉRIAUX INCORPORANT DES MICROPARTICULES POUR ÉVITER LA PROLIFÉRATION DE CONTAMINANTS**
VERWENDUNG VON MATERIALIEN MIT MIKROPARTIKELN ZUR VERMEIDUNG DER PROLIFERATION VON KONTAMINANTEN
USE OF MATERIALS INCORPORATING MICROPARTICLES FOR AVOIDING THE PROLIFERATION OF CONTAMINANTS

(30) Priorité: 25.06.2014 FR 1455871
(43) Date de publication de la demande: 03.05.2017
(73) Titulaire: Pylote, 31280 Dremil-Lafage (FR)
(72) Inventeur: MARCHIN, Loïc, 31280 Mons (FR)
(74) Mandataire: Cabinet Becker et Associés
(86) Numéro de dépôt international: PCT/FR2015/051730
(87) Numéro de publication internationale: WO 2015/197992

(56) Documents cités:
- WO-A1-2014/193875
- WO-A2-2012/104844
- US-A- 5 047 448
- US-A1- 2003 091 767
- US-A1- 2005 003 728
- US-A1- 2008 047 894
- US-A1- 2010 166 817
- US-A1- 2011 052 662
- US-B2- 7 169 402

## Description

### Introduction

La présente demande concerne l'utilisation d'un matériau solide comprenant une matrice dans laquelle sont dispersées des microparticules, lesdites particules ayant une activité antimicrobienne. La demande concerne également l'utilisation d'un tel matériau pour fabriquer un article, le procédé de fabrication dudit article, et l'article obtenu.

### Domaine technique

Les compositions, notamment cosmétiques ou pharmaceutiques, qui sont utilisées à plusieurs reprises sont sujettes à des risques de contamination due à leur exposition à l'air ambiant et/ou à un moyen d'application (un applicateur, un doigt, ...) et/ou à l'organe auquel la composition est destinée (par exemple l'oeil pour les collyres). Les emballages, contenants et/ou dispositifs de distribution de telles compositions peuvent également être sujets à contamination.

Pour éviter et/ou ralentir la contamination des compositions, la structure des emballages, contenants ou dispositifs de distribution de ces compositions est généralement adaptée pour isoler la partie de la composition non contaminée de la partie de la composition qui est en contact avec l'air ambiant, un moyen d'application, un organe et/ou toute autre source potentielle de contamination. Ainsi, les contenants adaptés pour contenir des compositions susceptibles d'être contaminées peuvent comprendre des moyens physiques d'obturation tels que des clapets, des valves et/ou des membranes permettant d'isoler chacune des deux parties de la composition l'une de l'autre. Ces contenants nécessitent un procédé de fabrication spécifique et élaboré, qui augmente notamment leur coût. En outre, de tels systèmes, s'ils évitent la contamination de la partie de la composition qui est isolée, n'évitent pas nécessairement la contamination de la partie de composition qui est délivrée, par exemple si l'embout servant à délivrer la composition est lui-même contaminé.

Alternativement, l'incorporation d'agents antimicrobiens organiques ou nanométriques dans le matériau constituant tout ou partie des emballages, contenants ou dispositifs de distribution a également été envisagée. Ainsi, des matrices plastiques comprenant des nanoparticules d'argent (nano-argent), des nanoparticules d'oxyde de zinc ou du triclosan (5-chloro-2-(2,4-dichlorophénoxy)phénol), un biocide organique, ont notamment pu être utilisées pour former des emballages, contenants ou dispositifs de distribution de compositions potentiellement sujettes à contamination. De par leur taille notamment, ces deux types d'agents (nanoobjets et composés organiques) migrent et se diffusent de façon non négligeable à partir de la matrice dans la composition. Une telle migration n'est pas désirée. D'une part, cette migration conduit à un « épuisement » du stock d'agent antibactérien contenu dans la matrice, ce qui contraint à incorporer une quantité importante de cet agent pour éviter que l'emballage, le contenant ou le dispositif de distribution ne perde ses propriétés antibactériennes. En outre, la toxicité suspectée ou avérée des antibactériens organiques et des nanoobjets dissuade d'utiliser des compositions les contenant, notamment dans les domaines cosmétique et pharmaceutique. Ainsi, le triclosan a notamment été identifié comme étant perturbateur endocrinien. Parmi les nanoparticules utilisées pour être incorporées dans des matrices plastiques, on peut citer notamment les nanoparticules d'oxyde de zinc commercialisées sous l'appellation Zano^{®} 20 par la société Umicore Zinc Chemicals.

Il serait intéressant de pouvoir disposer d'emballages, de contenants et/ou de dispositifs de distribution adaptés pour des compositions sujettes à contamination permettant d'éviter et/ou ralentir à la fois la contamination des parties des compositions qui ne sont pas en contact direct avec les sources de contamination (rétro-contamination), la contamination des parties de composition qui sont délivrées, la contamination des emballages, contenants et/ou dispositifs de distribution des compositions, et la migration d'agents antimicrobiens, initialement présents dans les emballages, contenants et/ou dispositifs de distribution, au sein des compositions. De préférence, ces emballages, contenants ou dispositifs devraient pouvoir être de conception mécanique simple, de préférence identique à celle utilisée pour des compositions ne risquant pas d'être contaminées, dont la fabrication est simplifiée et peu coûteuse.

Dans ce cadre, la Demanderesse a mis en évidence que l'incorporation de microparticules spécifiques dans des matrices, par exemples des matrices polymériques, permet de conférer aux matériaux obtenus des propriétés antimicrobiennes, sans que les agents antimicrobiens puissent migrer à l'extérieur des matériaux, notamment dans une composition mise en contact avec ces matériaux. Ces matériaux permettent également de supprimer et/ou ralentir la formation de biofilms à leur surface. Ces matériaux peuvent être utilisés notamment pour fabriquer des emballages, contenants ou dispositifs de distribution adaptés pour les compositions susceptibles d'être contaminées.

On connaît l'utilisation de particules antimicrobiennes dispersées dans une matrice divulguées dans les documents suivants : US 2003/091767, WO 2012/104844, US 2008047894, et US 5,047,448.

### Résumé de l'invention

Le premier objet de l'invention est l'utilisation d'un matériau solide comprenant une matrice et des microparticules comprenant ou constituées d'au moins un agent antimicrobien pour prévenir, limiter et/ou supprimer la contamination dudit matériau et/ou la contamination d'une composition qui est en contact avec ledit matériau pendant au moins un temps donné, et/ou prévenir, supprimer et/ou ralentir la formation de biofilms à la surface dudit matériau, dans laquelle l'agent antimicrobien ne migre pas à l'extérieur dudit matériau. Ladite utilisation et ledit matériau solide étant tels que définis dans les revendications.

Un second objet de l'invention est l'utilisation d'un matériau solide comprenant une matrice et des microparticules comprenant ou constituées d'au moins un agent antimicrobien pour fabriquer un article susceptible d'être mis en contact avec au moins une source de contamination microbienne, dans laquelle l'agent antimicrobien ne migre pas à l'extérieur du matériau ou de l'article. Ladite utilisation et ledit article étant tels que définis dans les revendications

Un autre objet de l'invention est un article constitué d'un matériau comprenant une matrice solide et des microparticules comprenant ou constituées d'au moins un agent antimicrobien, dans lequel l'article est susceptible d'être mis en contact avec au moins une source de contamination microbienne et dans lequel l'agent antimicrobien ne migre pas à l'extérieur du matériau ou de l'article. Ledit article étant tel que défini dans les revendications

Un dernier objet de l'invention est un procédé de fabrication d'un article susceptible d'être mis en contact avec au moins une source de contamination microbienne comprenant une étape de mise en forme d'un matériau solide comprenant une matrice et des microparticules comprenant ou constituées d'au moins un agent antimicrobien, dans lequel l'agent antimicrobien ne migre pas à l'extérieur du matériau ou de l'article. Ledit procédé de fabrication et ledit article étant tels que définis dans les revendications.

### Brève description des figures

- Figure 1 :: Cinétique de diminution de la prolifération pour une plaquette de matériau selon l'invention et pour une plaquette témoin (exemple 1).
- Figure 2 :: Evolution de la prolifération des bactéries en fonction du taux de conservateur pour une plaquette témoin (a) et pour une plaquette en matériau selon l'invention (b) (exemple 2).
- Figure 3 :: Comparaison des activités antibactériennes d'une plaquette témoin et d'une plaquette en matériau selon l'invention en fonction de la quantité de conservateur (exemple 2).

### Description détaillée de l'invention

Un premier objet de l'invention est l'utilisation d'un matériau solide comprenant une matrice et un ensemble de microparticules comprenant ou constituées d'au moins un agent antimicrobien pour prévenir, limiter et/ou supprimer la contamination dudit matériau et/ou la contamination d'une composition qui est en contact avec ledit matériau pendant au moins un temps donné, et/ou prévenir, supprimer et/ou ralentir la formation de biofilms à la surface dudit matériau, dans laquelle l'agent antimicrobien ne migre pas à l'extérieur dudit matériau.

Les microparticules de l'ensemble de microparticules compris dans le matériau utilisé selon la description peuvent être tout type de microparticules comprenant ou constituées d'au moins un agent antimicrobien. Il s'agit de microparticules sphériques. De préférence, l'ensemble de microparticules est un ensemble de microparticules individualisées et de préférence réparties uniformément dans la matrice, notamment au niveau de la surface du matériau qui est susceptible d'être en contact avec une source de contamination.

La demande concerne également un procédé pour prévenir, limiter et/ou supprimer la contamination d'un matériau et/ou la contamination d'une composition qui est en contact avec ledit matériau pendant au moins un temps donné, et/ou prévenir, supprimer et/ou ralentir la formation de biofilms à la surface dudit matériau, ledit procédé comprend la mise en oeuvre ou l'utilisation d'un matériau solide comprenant une matrice et un ensemble de microparticules, lesdites microparticules comprenant ou étant constituées d'au moins un agent antimicrobien. En particulier, le matériau permet audit agent antimicrobien de ne pas migrer à l'extérieur dudit matériau.

Par agent antimicrobien, on désigne une substance qui tue, ralentit la croissance ou bloque la croissance d'un ou de plusieurs microbes. Par « croissance », on désigne dans la présente invention toute opération cellulaire permettant un accroissement volumétrique de la cellule, une division cellulaire ou une reproduction cellulaire. Un microbe désigne selon la présente invention tout organisme unicellulaire ou pluricellulaire pathogène ou parasitaire d'autres organismes vivants comme les humains. Parmi les microbes, on peut citer notamment les moisissures, les champignons, les levures, les bactéries et les virus. Un agent antimicrobien selon la présente invention peut par exemple être un agent antibiotique, fongicide, fongistatique, bactéricide ou bactériostatique.

Par agent fongicide, fongistatique, bactéricide ou bactériostatique, on désigne un agent capable respectivement d'éliminer au moins un type de moisissure, champignon ou levure, de ralentir le développement d'au moins un type de moisissure, champignon ou levure, d'éliminer au moins un type de bactérie ou de ralentir le développement d'au moins un type de bactérie. L'agent fongicide, fongistatique, bactéricide ou bactériostatique des particules utilisées selon l'invention pourra être sélectionné par l'homme du métier en fonction des conditions d'utilisation mises en oeuvre et de l'effet que l'on cherche à obtenir.

Par bactérie, on désigne les eubactéries et les archéobactéries. Les eubactéries incluent les fermicutes, les gracilicutes et les ternicutes. Les gracilicutes incluent les bactéries Gram négatives telles que les Enterobacteriaceae, comme par exemple Klebsellia (telle que *Klebsellia pneumoniae*) et Escherichia (telle que *Escherichia coli*). Les fermicutes incluent les bactéries Gram positif, telles Micrococcaceae, comme par exemple les Staphylocoques (tel que *Staphylococcus aureus*) et les tiges formant des endospores incluant les bacilles (Bacillaceae) comme par exemple *Bacillus circulans.* Toutes ces références sont mentionnées dans le Bergey's Manual of Systematic Bacteriology, Williams & Wilkens, 1st ed. Vol. 1-4, (1984). Par moisissures, on désigne notamment les champignons et levures.

Par champignons, on désigne les champignons ou mycètes présents dans un environnement. Le terme myctes (Myceteae) inclut Amastigomycota comme par exemple Deuteromycotina, qui inclut les Deuteromycetes. Les Deuteromycetes incluent Aspergillis (*Aspergillus niger*) et Candida (*Candida albicans*).

Par biofilm, on désigne dans la présente invention une communauté multicellulaire de microorganismes (bactéries, champignons, algues ou protozoaires par exemple), adhérant entre eux et à une surface du matériau, et marquée par la sécrétion d'une matrice adhésive et protectrice.

Dans un mode de réalisation de la demande, les particules sont des particules d'un oxyde (par exemple monoxyde ou dioxyde) d'un ion métallique chargé positivement (Mⁿ⁺, avec n entier compris entre 1 et 4), en particulier doublement chargé positivement (M²⁺), plus spécifiquement l'oxyde métallique n'est pas l'oxyde de cuivre. Par exemple, il peut s'agir de particules d'oxyde de zinc, d'oxyde de magnésium, de dioxyde de titane, un mélange de telles particules, ou des particules comprenant plusieurs de ces oxydes. En particulier, il peut s'agir de particules comprenant des nanoparticules d'un tel oxyde dans une matrice telle qu'une matrice de silice amorphe.

Selon l'invention, il s'agit de particules comprenant de l'oxyde de zinc ou constituées d'oxyde de zinc (ZnO) ou comprenant de l'oxyde de magnésium ou constituées d'oxyde de magnésium (MgO), ou un mélange d'oxyde de magnésium et d'oxyde de zinc. Dans un mode de réalisation plus spécifique, il s'agit de particules comprenant de l'oxyde de zinc ou constituées d'oxyde de zinc (ZnO).

Ces particules d'oxyde de zinc ou de magnésium ou un mélange des deux peuvent être en outre constituées de dioxyde de titane. Le dioxyde de titane peut être compris en une proportion de 10% en masse au maximum, de préférence 5% en masse au maximum, en particulier 2% en masse au maximum, par rapport à la masse totale de particules.

Les particules, en particulier les particules d'oxyde métallique, selon l'invention peuvent être dopées par au moins un élément chimique dit dopant. Le dopant est de préférence adapté pour augmenter et/ou optimiser les propriétés de ralentissement et/ou de suppression de la prolifération de contaminants, de préférence les propriétés fongicides, fongistatiques, bactéricides ou bactériostatiques, des particules d'oxyde métallique. Par exemple, les particules d'oxyde de zinc peuvent être dopées par au moins un ion chargé positivement (D^{m+} avec m entier compris entre 1 et 4), en particulier par du calcium, sodium, magnésium, titane et/ou de l'aluminium.

Le dopant est compris en une proportion de 10% en masse au maximum, de préférence 5% en masse au maximum, en particulier 2% en masse au maximum, par rapport à la masse totale de particules.

Les particules peuvent comprendre, en plus de la substance ayant des propriétés antimicrobiennes, un autre composé ayant des propriétés particulières. Par exemple, les particules peuvent comprendre en outre un ingrédient actif, tel qu'une huile essentielle.

Dans un mode de réalisation, les microparticules sont des particules mésoporeuses, encapsulant éventuellement un composé ayant des propriétés particulières, de préférence antimicrobiennes, tel qu'une huile essentielle. Dans un mode de réalisation, les microparticules mésoporeuses sont des particules d'un oxyde d'ion métallique telles que définies ci-avant encapsulant un composé tel qu'une huile essentielle.

Les particules comprises dans le matériau utilisé sont des microparticules, c'est-à-dire que leur diamètre moyen (en nombre) est compris entre 0,1 et 1000 micromètres. Selon l'invention, les particules ont un diamètre moyen compris entre 0,1 et 5 micromètres, de préférence entre 0,4 et 5 micromètres, notamment entre 0,5 et 3 ou entre 0,5 et 2 micromètres, en particulier un diamètre moyen d'environ 0,5 micromètres. L'homme du métier connaît les techniques adaptées pour déterminer la valeur du diamètre des particules ou des ensembles de particules selon l'invention. Selon l'invention, le diamètre moyen des particules d'un ensemble, l'écart-type et la distribution des tailles notamment sont déterminés par des études statistiques à partir d'images de microscopie, par exemple de microscopie électronique à balayage (MEB) ou en transmission (MET).

Le terme « environ » relatif à une valeur numérique désigne dans la présente demande un intervalle centré sur la valeur numérique, et s'étendant de 10% au-dessus et en-dessous de cette valeur.

Dans la présente invention, un ensemble de particules individualisées désigne un ensemble de particules dans lequel les particules ne sont pas agrégées, c'est-à-dire que chaque particule de l'ensemble n'est pas liée à d'autres particules par des liaisons chimiques fortes telles que des liaisons covalentes.

Un ensemble de particules utilisées selon l'invention peut éventuellement contenir de façon ponctuelle des particules ne répondant pas à cette caractéristique, dans la mesure où le critère de non agrégation est respecté par au moins 50% en nombre des particules de l'ensemble. De préférence, au moins 60%, au moins 70%, au moins 80%, au moins 90%, au moins 95% en nombre des particules de l'ensemble considéré sont individualisées.

De préférence, une particule utilisée selon l'invention n'est pas constituée par l'agrégation de plusieurs particules de taille inférieure. Ceci peut être clairement visualisé par exemple par des études en microscopie, notamment en microscopie électronique à balayage (MEB) ou en transmission (MET). Ceci signifie que les seuls constituants possibles des particules selon l'invention sont des cristallites de taille nettement inférieure à celle des particules selon l'invention. Une particule selon l'invention est de préférence formée d'au moins deux cristallites. Une cristallite est un domaine de matière ayant la même structure qu'un monocristal, c'est-à-dire qu'au sein de chaque plan atomique définissant cette structure il n'y a pas de discontinuité majeure de l'ordre cristallin hormis des défauts ponctuels (lacunes, atomes en insertion ou substitution) ou linéaires (dislocations).

De préférence, les particules utilisées selon l'invention sont individualisées et non déformables. Aussi, la surface de chaque particule qui est en contact avec d'autres particules est généralement très faible. Dans un mode de réalisation, le rayon de courbure du ménisque formant le contact entre deux particules différentes de l'ensemble est inférieur à 5%, de préférence inférieur à 2%, du rayon de chacune des deux particules, en particulier au sein d'une matrice selon l'invention.

Les particules sont sphériques, en particulier elles présentent un coefficient de sphéricité supérieur ou égal à 0,75. De préférence, le coefficient de sphéricité est supérieur ou égal à 0,8, supérieur ou égale à 0,85, supérieur ou égal à 0,9, ou encore supérieur ou égal à 0,95. Selon l'invention, les particules sont sphériques et présentent un coefficient de sphéricité moyen en nombre supérieur ou égal à 0,9.

Le coefficient de sphéricité d'une particule est le rapport du plus petit diamètre de la particule au plus grand diamètre de celle-ci. Pour une sphère parfaite, ce rapport est égal à 1. Le coefficient de sphéricité peut être calculé par exemple par mesure du rapport d'aspect au moyen de tout logiciel adapté à partir d'images, par exemple d'images obtenues par microscopie, en particulier microscopie électronique à balayage ou en transmission, des particules.

Un ensemble de particules utilisées selon l'invention peut éventuellement contenir de façon ponctuelle des particules n'ayant pas les critères requis de sphéricité dans la mesure où la sphéricité moyenne en nombre sur l'ensemble des particules répond aux critères fixés dans la présente invention. De préférence, au moins 50%, au moins 60%, au moins 70%, au moins 80%, au moins 90%, au moins 95% en nombre des particules de l'ensemble considéré ont une sphéricité telle que définie ci-dessus.

Les particules micrométriques, en particulier selon les tailles telles que définies ci-dessus, présentent plus spécifiquement des surfaces spécifiques élevées. Selon un mode particulier, les particules selon l'invention présentent des surfaces spécifiques supérieures ou égales à 15m²/g, de préférence supérieures ou égales à 30m²/g. Les surfaces spécifiques des particules selon l'invention peuvent aller jusqu'à 700 m²/g ou 600 m²/g. Bien entendu, les surfaces spécifiques varient, notamment en fonction du diamètre des particules et de leur porosité. Selon un mode particulier, le diamètre moyen des particules selon l'invention est compris entre 0,2 et 5 micromètres et de préférence entre 0,4 et 3 micromètres, et présentent des surfaces spécifiques supérieures ou égales à 15m²/g, de préférence supérieures ou égales à 30m²/g. Les surfaces spécifiques peuvent être mesurées selon différentes méthodes, en particulier selon la méthode Brunauer, Emmett, Teller (BET) ou la méthode Barrett, Joyner, Halenda (BJH). Les valeurs de surfaces spécifiques spécifiées ci-dessus sont données selon la méthode BET, sauf mention contraire.

Dans un mode de réalisation particulier, les particules sont telles que décrites dans la demande de brevet français déposée le 7 mai 2014 sous le numéro FR 14 54141 ou la demande de brevet PCT n° PCT/FR2015/051223 déposée le 7 mai 2015.

Les particules individualisées utilisées selon l'invention peuvent être préparées par n'importe quel procédé connu de l'homme du métier. En particulier, elles peuvent être préparées par le procédé décrit dans la demande de brevet français déposée sous le numéro FR 14 54141 ou la demande de brevet PCT n° PCT/FR2015/051223.

Cette demande décrit un procédé de préparation d'un ensemble de particules dit « par pyrolyse d'aérosol » (ou spray pyrolyse) qui est mis en oeuvre à des températures de séchage et pas nécessairement de pyrolyse., En particulier, ce procédé comprend les étapes successives suivantes :
- (1) la nébulisation d'une solution liquide contenant un précurseur du ou des matériaux inorganiques duquel ou desquels on veut former des particules à une concentration molaire donnée dans un solvant, de sorte à obtenir un brouillard de gouttelettes de solution,
- (2) le chauffage du brouillard à une température (dite de séchage) apte à assurer l'évaporation du solvant et la formation de particules,
- (3) le chauffage de ces particules à une température (dite de pyrolyse) apte à assurer la décomposition du précurseur pour former le matériau inorganique,
- (4) optionnellement la densification des particules,
- (4') optionnellement la trempe des particules, et
- (5) la récupération des particules ainsi formées.

Plus précisément, le procédé de préparation d'un ensemble de particules selon l'invention est généralement mis en oeuvre dans un réacteur. L'ensemble de particules ainsi obtenu peut ainsi correspondre à des grandes quantités, plus spécifiquement la quantité obtenue par jour peut être de plus de 100g, 500g, 1kg, 15 kg, ou 20kg, cette quantité variant en fonction de l'alimentation en solution fournie et/ou désirée au réacteur. L'ensemble des particules ainsi obtenu présente donc l'avantage d'être obtenu en grande quantité, tout en respectant les caractéristiques des particules telles que décrites ci-avant.

L'étape (1) de nébulisation est réalisée de préférence à une température de 10 à 40 °C, et/ou de préférence pendant une durée inférieure ou égale à 10 secondes, en particulier inférieure ou égale à 5 secondes. A l'étape (1), la solution liquide est en général sous forme de solution aqueuse ou hydro alcoolique ou sous forme d'un sol colloïdal. Plus spécifiquement, la solution liquide de l'étape (1) est introduite dans un réacteur par nébulisation.

L'étape (2) de chauffage (séchage) est réalisée de préférence à une température de 40 à 120 °C, et/ou de préférence pendant une durée inférieure ou égale à 10 secondes, en particulier comprise entre 1 et 10 secondes.

L'étape (3), dite de pyrolyse, est réalisée de préférence à une température de 120 à 400 °C, et/ou de préférence pendant une durée inférieure ou égale à 30 secondes, en particulier comprise entre 10 et 30 secondes.

L'étape (4) optionnelle de densification peut être réalisée dans une large gamme de températures, notamment entre 200 et 1000°C. Cette étape est réalisée de préférence à une température de 400 à 1000 °C, en particulier lorsque les particules que l'on veut préparer sont au moins en partie sous forme cristallisée. Lorsque l'on cherche à obtenir des particules denses mais non cristallisées, en particulier des particules amorphes, la température de densification peut être plus faible, par exemple elle peut être aux alentours de 200°C à 300°C, notamment pour la silice amorphe. De préférence, l'étape de densification est réalisée pendant une durée inférieure ou égale à 30 secondes, en particulier comprise entre 20 et 30 secondes.

L'étape (5) de récupération est réalisée de préférence à une température inférieure à 100 °C, et/ou de préférence pendant une durée inférieure ou égale à 10 secondes, en particulier inférieure ou égale à 5 secondes. L'étape (5) de récupération des particules est réalisée de préférence par dépôt des particules sur un filtre en sortie du réacteur.

Les températures de chacune des étapes peuvent se situer en dehors des gammes de températures fournies ci-dessus. En effet, pour les mêmes particules, la température à appliquer pourra dépendre de la vitesse à laquelle les gouttes puis les particules circulent dans le réacteur. Plus les gouttes puis les particules circulent vite dans le réacteur, moins elles y passent de temps et plus la température de consigne doit être élevée pour obtenir le même résultat.

De préférence, les étapes (2), (3) et (4) sont réalisées dans le même réacteur. En particulier, l'ensemble des étapes du procédé (excepté les étapes de post-traitement) sont réalisées dans le même réacteur.

L'ensemble des étapes du procédé, en particulier les étapes (2), (3) et (4), sont réalisées dans la continuité l'une de l'autre. Le profil de température appliqué dans le réacteur est adapté en fonction des particules que l'on souhaite former pour que ces trois étapes aient lieu les unes après les autres. De préférence, la température dans le réacteur est ajustée par l'intermédiaire d'au moins un, de préférence 3, éléments chauffants dont les températures peuvent être définies indépendamment.

Le procédé de préparation d'un ensemble de particules selon la présente invention comprend de préférence en outre entre l'étape (3), ou éventuellement l'étape de densification des particules (4) lorsqu'elle est mise en oeuvre, et l'étape de récupération des particules (5) une étape (4') de trempe des particules. L'étape de trempe (4') correspond à une diminution en température rapide. Plus spécifiquement, lorsque l'étape de densification des particules (4) est mise en oeuvre, l'étape de trempe a de préférence lieu, et elle correspond avantageusement à une diminution de température d'au moins 300°C/s, par exemple pour atteindre une température comprise entre 15 et 50°C. Plus spécifiquement, lorsque l'étape de densification des particules (4) n'est pas mise en oeuvre, l'étape de trempe a éventuellement lieu, et, si elle a lieu, elle correspond avantageusement à une diminution de température d'au moins 100°C/s. L'étape de trempe (4') est de préférence réalisée par entrée d'un gaz, de préférence de l'air, froid sur tout ou partie de la circonférence du réacteur. Un gaz est dit froid dans la présente invention s'il est à une température comprise entre 15 et 50°C, de préférence entre 15 et 30°C. Dans un mode de réalisation, le gaz entrant dans le réacteur est un gaz différent de l'air. En particulier, il peut s'agir d'un gaz neutre (tel que l'azote ou l'argon), d'un gaz réducteur (tel que l'hydrogène ou le monoxyde de carbone), ou d'un quelconque mélange de tels gaz.

Le procédé de préparation d'un ensemble de particules est mis en oeuvre de préférence en absence de flux de gaz vectorisant le brouillard depuis le début (e.g. le bas) du réacteur. Le flux laminaire permettant d'amener la matière dans la zone dans laquelle la température est plus élevée est avantageusement créé uniquement par l'aspiration en fin (e.g. haut) du réacteur, produisant une dépression, par exemple de l'ordre de quelques pascals ou quelques dizaines de pascals.

Un tel mode de réalisation permet d'utiliser un réacteur sans entrée de gaz dans sa partie inférieure, limitant ainsi les perturbations du procédé et les pertes, et optimisant ainsi le rendement du procédé et la distribution en taille des particules obtenues.

Dans un autre mode de réalisation, le réacteur dans lequel le procédé est mis en oeuvre comprend également une entrée de gaz au niveau où le brouillard est formé. Le gaz qui entre dans le réacteur à ce niveau est de préférence de l'air, en particulier de l'air chaud, c'est-à-dire à une température de 80 à 200°C.

De préférence, le procédé selon l'invention ne comprend pas d'autre étape de chauffage que celles mises en oeuvre à l'intérieur du réacteur de pyrolyse d'aérosol.

Le précurseur ou les précurseurs du ou des matériaux inorganiques duquel ou desquels on veut former des particules (en particulier d'un oxyde d'un ion métallique chargé positivement, comme ZnO ou MgO) peut être de toute origine. Il(s) est(sont) introduit(s) à l'étape (1) du procédé sous forme d'une solution liquide, en particulier une solution aqueuse ou hydro alcoolique contenant les ions métalliques (en particulier un sel organique ou minéral du métal considéré) ou les molécules précurseurs (comme des organosilanes) ou encore sous forme d'un sol colloïdal (comme une dispersion colloïdale de nanoparticules du métal ou de l'oxyde du métal considéré). De préférence, le précurseur ou les précurseurs du ou des matériaux inorganiques est(sont) introduit(s) à l'étape (1) du procédé sous forme d'une solution liquide, en particulier une solution aqueuse ou hydro alcoolique contenant les ions métalliques (comme un sel organique ou minéral du métal considéré). Le ou les précurseurs du ou des matériaux inorganiques est ou sont choisi(s) en fonction des particules que l'on souhaite former.

Les matériaux selon l'invention ont prouvé une grande efficacité anti-contamination alors que les taux de particules incorporées dans la matrice sont faibles. L'efficacité anticontamination peut notamment être mesurée selon la norme ISO22196 (ou JIS Z 2801) qui permet d'évaluer l'action antibactérienne sur des surfaces en plastique et autres surfaces non poreuses. Ainsi, selon un aspect particulier, des matériaux selon l'invention ont été testés selon cette norme et présentent une activité antibactérienne comprise entre 0 et 7 UFC/cm², ou comprise entre 1 et 7 UFC/cm², en particulier comprise entre 2 et 7 UFC/cm², plus particulièrement comprise entre 4 et 5,3 UFC/cm². L'homme du métier adaptera les critères d'activité antimicrobienne spécifique en fonction de l'application envisagée.

Des taux de particules faibles sont connus comme étant adaptés pour éviter que les particules ne migrent hors du matériau. La combinaison de l'efficacité anti-contamination et de l'absence de relargage des particules des matériaux selon l'invention peut permettre de diminuer le taux de conservateurs, voire de s'en affranchir, au sein des compositions, notamment alimentaires et/ou pharmaceutiques, dermatologiques ou cosmétiques, qui sont en contact avec ces matériaux. Ainsi, il été démontré que les matériaux selon l'invention permettent dans certaines conditions de diminuer d'un facteur 4 le taux de conservateurs dans une composition en contact avec la matrice plastique pour obtenir le même taux de prolifération bactérienne. La combinaison de l'efficacité anti-contamination et de l'absence de relargage des particules des matériaux selon l'invention peut de même permettre de retarder la date de péremption de la composition. Les propriétés des matériaux selon l'invention peuvent également permettre de diminuer les doses de radiation, par exemple de radiation gamma, utilisées pour décontaminer et/ou stériliser les articles formés avec ces matériaux. Il est bien connu en effet que la stérilisation par irradiation présente des inconvénients. Par exemple, elle peut contribuer à décolorer et/ou conférer des odeurs à un emballage et/ou à une composition cosmétique.

La matrice utilisée pour préparer le matériau selon l'invention est avantageusement une matrice liquide, quelle que soit sa viscosité, qui permet, après incorporation de l'ensemble de microparticules dans la matrice, et éventuellement après une étape additionnelle telle qu'une étape de séchage, de former un matériau solide qui peut être utilisé selon l'invention. La caractéristique « liquide » de la matrice peut éventuellement être obtenue par une action, par exemple par chauffage, d'une matrice qui n'est pas liquide. L'incorporation des microparticules dans la matrice est réalisée de préférence lorsque la matrice est sous forme liquide ou fondue, une fois les microparticules incorporées, la matrice est rendue solide afin d'avoir un matériau solide.

Selon la demande, la matrice est une matrice inorganique ou organique. Selon l'invention la matrice est une matrice organique par exemple de la famille polymérique, en particulier une matrice polymérique de type plastique, caoutchouc, vernis, peinture, textile, silicone, colle, revêtements, élastomère. Selon un aspect particulier, la matrice polymérique est constituée de polymères thermoplastiques comme notamment le copolymère acrylonitrile butadiène styrène, l'acétate de cellulose, le polystyrène, notamment le polystyrène expansé, les polyamides, le poly(téréphtalate de butylène), les polycarbonates, le polyéthylène haute densité, le polyéthylène basse densité, le poly(téréphtalate d'éthylène), le poly(méthacrylate de méthyle), le polyformaldéhyde, le polypropylène, le poly(acétate de vinyle), le poly(chlorure de vinyle), le poly(acide lactique) (PLA), la polycaprolactone, le polyhydroxyalcanoate (PHA), les polysaccharides, le copolymère styrène-acrylonitrile, ou un mélange de tels polymères.

Dans un mode de réalisation particulier, la matrice polymère est une matrice d'un biopolymère, c'est-à-dire d'un polymère issu de la biomasse, autrement dit produit par des êtres vivants tels que des végétaux, algues, animaux ou champignons par exemple.

La matrice peut également être une peinture, une encre, ou une matrice précurseur d'un matériau textile ou de n'importe quel matériau apte à former des films et/ou revêtements sur des surfaces. Par matériau textile, on peut citer notamment les matériaux adaptés pour les vêtements, les tapis, les rideaux, les literies et les matériaux textiles médicaux tels que les pansements.

La proportion de microparticules réparties dans le matériau peut varier dans une large mesure en fonction de la nature des particules et de la matrice, et de l'utilisation envisagée pour le matériau et/ou l'article. De préférence, le matériau ou l'article selon l'invention comprend un faible taux de particules par rapport à la matrice, en particulier de 0,1 à 10%, ou de 0,1 à 5%, plus spécifiquement de 0,5 à 3%, ou de 1 à 3%, de particules en masse par rapport à la masse totale (masse de la matrice et de particules). De façon hautement préférée, le matériau ou l'article selon l'invention comprend d'environ 0,2 à 2,5% en masse de particules par rapport à la masse totale (masse de la matrice et de particules). L'homme du métier est à même d'ajuster la proportion de particules dans le matériau pour obtenir à la fois l'effet anticontamination, en particulier l'effet antimicrobien, désiré et un taux de relargage (migration des particules hors du matériau) aussi faible que possible. Bien entendu, il sera d'autant plus facile de maintenir un taux de relargage faible que la quantité de particules dans le matériau sera faible. Les matériaux selon l'invention ont la particularité de conserver des taux de relargage très faibles (voire nuls), ce qui correspond à une migration très faible (voire nulle) des particules lorsque les proportions de particules sont suffisantes pour obtenir un effet antimicrobien notable.

Le matériau utilisé selon l'invention possède ainsi des propriétés de suppression et/ou ralentissement de la prolifération de contaminants. Ces propriétés sont d'autant plus efficaces que la composition et/ou l'objet susceptible d'être contaminé est placé proche dudit matériau. En particulier, la composition et/ou l'objet susceptible d'être contaminé est placé au contact du matériau afin de ralentir et/ou supprimer la prolifération des contaminants.

Les propriétés conférées au matériau par l'inclusion des particules selon l'invention dans la matrice ne diminuent pas ou très peu dans le temps, car il n'y a aucun transfert des particules à la composition et/ou l'objet. Par exemple, l'action bactéricide des particules d'oxyde de zinc est obtenue par création d'espèces réactives de l'oxygène, qui tuent les bactéries, lorsque les particules sont au contact du dioxygène de l'air. Il n'y a aucune consommation des particules d'oxyde de zinc mais uniquement consommation du dioxygène de l'air ou de l'environnement dans lequel le matériau se trouve.

Dans la présente invention, le fait de ne pas avoir de migration des microparticules hors du matériau, en particulier aucun transfert des microparticules à la composition qui est en contact avec le matériau, signifie qu'il y a moins de 1 mg, de préférence moins de 0,5 mg, en particulier moins de 0,01 mg, de l'élément principal constituant les microparticules qui se retrouve dans 1 kilogramme de composition. L'élément principal constituant les microparticules est l'oxyde métallique tel que défini ci-dessus, en particulier l'oxyde de magnésium et/ou l'oxyde de zinc. En particulier, ce taux peut être mesuré par la méthode suivante, et préconisée par la pharmacopée européenne (par exemple : chapitres 3.1.3 pour les polyoléfines, 3.1.5 pour les polyéthylènes, ou 3.1.6 pour les polypropylènes) : Les échantillons de matériaux sont préalablement découpés en morceaux de 1cm de côté au maximum. Dans une fiole conique de verre borosilicaté à col rodé, 100g du matériau selon l'invention à examiner sont introduits. 20mL d'acide chlorhydrique 0.1M sont ajoutés. L'ensemble est chauffé à reflux pendant 1h en maintenant une agitation constante. La solution est refroidie à température ambiante (i.e. entre 18 et 25°C) et laissée à décanter. Les extractibles sont mesurés par spectrométrie d'absorption atomique. Les essais ont permis de mesurer un taux d'extractibles ≤ à 1 ppm dans ces conditions d'essais pour le polypropylène comme matrice (l'extractible étant le zinc, quand les microparticules sont à base de Zn0).

Ainsi, lorsque l'agent antimicrobien selon l'invention est un oxyde métallique sous forme de microparticules, les termes « l'agent antimicrobien ne migre pas à l'extérieur dudit matériau » peuvent correspond à un taux de migration inférieur ou égal à 50 ppm, à 25 ppm, en particulier inférieur ou égal à 10 ppm, ou plus spécifiquement inférieur à 5ppm, d'ion métallique (la limite inférieure étant généralement comprise entre 0 et 1 ppm d'ion métallique). Les conditions de ces tests sont bien entendu des conditions plus drastiques que les conditions d'usage rencontrées pour les matériaux en conditions normales d'usage.

Bien entendu, un taux de migration sortant de cette gamme peut éventuellement être observé dans des situations très particulières, notamment lorsque le matériau est placé dans des conditions le dégradant, par exemple des conditions très acides. Néanmoins, de telles conditions ne sont généralement pas compatibles avec les compositions alimentaires, cosmétiques, dermatologiques ou pharmaceutiques.

Par conséquent, les matériaux utilisés selon l'invention conservent leurs propriétés de suppression et/ou ralentissement de la prolifération des contaminants pendant un temps plus long que ceux contenant des agents antimicrobiens qui peuvent migrer hors du matériau. De préférence, le matériau conserve ses propriétés pour une durée supérieure à la durée de conservation de la composition qui est en contact avec le matériau. En particulier, le matériau conserve ses propriétés tout au long de sa vie.

De préférence, la composition qui est en contact avec le matériau selon l'invention est une composition alimentaire, diététique, cosmétique, dermatologique ou pharmaceutique. De préférence, il s'agit d'un liquide tel qu'une solution ophtalmique, d'une crème telle qu'une crème cosmétique ou dermatologique, d'un gel, ou d'un produit alimentaire. Ainsi, selon un aspect particulier de l'invention, la composition est une composition physiologiquement acceptable pour un mammifère, en particulier un être humain, c'est-à-dire qu'elle n'engendre pas de fonctions ou de réactions anormales de l'organisme dudit mammifère ; aucune innocuité n'a été constatée pour une composition physiologiquement acceptable pour un mammifère. Le temps donné pendant lequel la composition est en contact avec le matériau peut varier dans une large mesure. Ainsi, lorsque le matériau est utilisé pour fabriquer un dispositif de distribution d'une crème cosmétique par exemple, la composition peut rester en contact avec le matériau pendant plusieurs semaines, plusieurs mois, voire plusieurs années. Lorsque le matériau est utilisé pour fabriquer ou revêtir des conduits acheminant des compositions alimentaires, la composition peut rester en contact avec le matériau pendant des temps beaucoup plus courts, de l'ordre de la minute ou de la seconde, voire inférieurs à une seconde.

L'utilisation d'un matériau selon l'invention permet de maintenir la composition contenue dans l'article formé du matériau selon l'invention propre, c'est-à-dire exempte de toute contamination microbienne ayant pour origine son exposition, de préférence son exposition répétée, à l'air ambiant et/ou à un moyen d'application et/ou à l'organe auquel la composition est destinée.

Par « exposition répétée », on désigne le fait que l'article est utilisé au moins deux fois pour délivrer au moins une partie de la composition, et que par conséquent une partie de la composition reste en contact avec l'article après la première utilisation.

En outre, l'orifice permettant de délivrer la composition, par exemple l'extrémité de sortie de la pompe ou d'un tube, reste propre malgré sa mise en contact avec l'air ambiant, un moyen d'application et/ou un organe. Ainsi, il n'y a pas de contamination de la partie de la composition qui sera délivrée lors de l'utilisation suivante via cet orifice, au contraire des systèmes précédemment présentés tels que les systèmes à membranes ou à clapets dont les orifices peuvent être contaminés.

Dans le cas des produits alimentaires, l'utilisation d'un contenant selon l'invention permet notamment de limiter la prolifération microbienne notamment en surface du produit alimentaire, et ainsi de retarder, voire supprimer, la date limite de consommation dont la détermination prend généralement en compte le risque de prolifération bactérienne au niveau du produit alimentaire.

Un autre objet de l'invention est l'utilisation d'un matériau comprenant une matrice et des microparticules telles que définies ci-avant pour fabriquer un article susceptible d'être mis en contact avec au moins une source de contamination microbienne.

Par « source de contamination microbienne », on désigne dans la présente invention n'importe quel élément susceptible de contenir des microbes et susceptible de les transmettre au matériau, à la composition et/ou à l'article tels que définis dans la présente invention.

Dans un mode de réalisation, la source de contamination microbienne est choisie parmi l'air ambiant, un moyen d'application, tel qu'un pinceau ou une spatule, et un organe du corps humain ou animal.

Un autre objet de l'invention est un procédé de fabrication d'un article susceptible d'être mis en contact avec au moins une source de contamination microbienne comprenant une étape de mise en forme d'un matériau solide comprenant une matrice et des microparticules comprenant ou constituées d'au moins un agent antimicrobien tel que décrit ci-avant.

Le procédé de fabrication peut comprendre une étape préalable de dispersion des microparticules dans la matrice. Cette dispersion peut être réalisée par simple mélange, éventuellement sous agitation mécanique ou magnétique, ou sous sonication.

Le procédé de fabrication comprend donc une étape de mise en forme de l'article par n'importe quelle technique connue de l'homme de l'art et adaptée pour la mise en forme de la matrice et/ou du matériau. Ainsi, par exemple, dans le cas d'une matrice polymérique, l'étape de mise en forme peut être réalisée par moulage par injection, par injection-étirage-soufflage, ou par extrusion-soufflage.

Un autre objet selon l'invention est un article formé ou constitué d'un matériau solide comprenant une matrice et des microparticules comprenant ou constituées d'au moins un agent antimicrobien tel que défini ci-avant, dans lequel l'article est en particulier susceptible d'être mis en contact avec au moins une source de contamination microbienne.

L'article est tout ou partie d'un emballage, un contenant ou un dispositif de distribution d'une composition alimentaire, diététique, cosmétique, dermatologique ou pharmaceutique. L'article selon l'invention peut être à usage unique ou à multiusage.

L'article selon l'invention peut ainsi être notamment choisi parmi des bouchons, des opercules, des joints, des capsules, des couvercles, des bondes et des robinets destinés à la fermeture de bouteilles, de flacons, de pots, de boîtes, de bidons, de barriques, de cuves et de divers récipients utilisés pour le conditionnement et/ou le stockage de produits alimentaires, diététiques, cosmétiques, dermatologiques ou pharmaceutiques.

Alternativement, l'article peut être tout ou partie de bouteilles, de flacons, de pots, de boîtes, de bidons, de barriques, de cuves et de divers récipients utilisés pour le conditionnement et/ou le stockage de produits alimentaires, diététiques, cosmétiques, dermatologiques ou pharmaceutiques.

Dans un mode de réalisation préféré, l'article est un contenant et/ou un dispositif de distribution d'une composition, en particulier une solution ophtalmique, telle qu'un collyre ou du produit à lentilles. Il s'agit avantageusement d'un flacon à usage unique ou à multi-usage, éventuellement à visée pharmaceutique. Par exemple, l'homme du métier connaît bien les dispositifs de distribution d'un produit ophtalmique 3 pièces. Une, deux ou trois des trois pièces du dispositif peuvent être réalisées avec un matériau selon l'invention.

Parmi les articles adaptés pour la conservation et/ou la distribution de produits pharmaceutiques, on peut citer notamment les cuillers (comme les cuillers de sirop), les seringues (comme les seringues pour administrer par exemple un sirop), les plaquettes (comme les plaquettes de comprimés ou gélules), les poches (comme les poches de perfusion), les tubes, canules, pompes et flacons.

Parmi les articles adaptés pour la conservation et/ou la distribution de produits alimentaires, on peut citer les barquettes, opercules et films d'emballage.

Parmi les articles susceptibles d'être en contact avec des sources de contamination, on peut également mentionner la tuyauterie, les canalisations et les plans de travail.

La forme des emballages, contenants ou dispositifs de distribution d'une composition alimentaire, diététique, cosmétique, dermatologique ou pharmaceutique est bien connue dans l'art. Par exemple, les dispositifs de distribution d'une solution comprennent généralement un corps substantiellement cylindrique moulé (incluant les corps ovales) ayant un fond et un bec ou une canule, et un bouchon refermable, notamment un bouchon à vis, sur sa partie supérieure.

Un matériau ou un article selon l'invention peut être utilisé dans une large variété d'utilisations et de domaines. Par exemple, un tel matériau ou article peut être utilisé pour fabriquer et/ou revêtir des conduits acheminant des constituants susceptibles d'être contaminés, notamment au sein d'un site industriel. Ainsi, par exemple, le matériau ou l'article peut être utilisé pour fabriquer des conduits de transports d'aliments ou de produits alimentaires dans les usines agroalimentaires. L'utilisation d'un matériau ou d'un article selon l'invention permet notamment dans ce cas de limiter la formation de biofilms et l'apparition et/ou la prolifération de germes. Ceci permet d'éviter ou de réduire la fréquence des lavages des conduits, qui impliquent souvent des techniques spécifiques et nécessitent l'immobilisation des conduits pendant une durée non négligeable, ce qui peut impacter la productivité de l'usine. En outre, l'utilisation d'articles ou de matériaux selon l'invention permet d'obtenir l'effet désiré sur toute la surface, même au niveau des zones cachées ou par exemple des recoins difficilement accessibles au lavage.

De façon similaire, les articles et/ou matériaux selon l'invention peuvent être utilisés pour fabriquer des équipements pour n'importe quel environnement susceptible d'être contaminé, par exemple un équipement de bloc opératoire, un équipement de pièce stérile, un instrument chirurgical, ou une paillasse (plan de travail) de laboratoire.

Les exemples ci-après sont fournis à titre illustratif, et non limitatif, de la présente invention.

### EXEMPLES

### Exemple 1 : Evaluation de l'effet antibactérien de matériaux selon l'invention

La prolifération de différents types de bactéries (*Escherichia coli, Staphylococcus aureus, Pseudomonas aeruginosa, Listeria monocytogenes, Salmonella enterica, Staphylococcus epidermidis, Streptococcus pneumoniae et Haemophilus influenzae*) a été étudiée à la surface de plaquettes de matériau selon l'invention et comparée à celle obtenue pour des plaquettes équivalentes ne comprenant pas de particules. L'essai a été réalisé selon la norme JIS Z 2801 :2010. Les essais sont réalisés en triplicat, les résultats fournis ci-dessous correspondant à la moyenne sur les trois échantillons.

Les souches *Listeria monocytogenes, Salmonella enterica* sont classiquement associées au développement de bactéries pour les produits alimentaires.

Les souches *Staphylococcus epidermidis, Streptococcus pneumoniae et Haemophilus influenzae* sont classiquement associées au développement de bactéries au niveau nasal, donc susceptibles de se retrouver sur tout article qui entre en contact avec le visage.

L'échantillon selon l'invention est une plaquette de polyéthylène basse densité (Purell PE 1840 H commercialisé par la société Lyondelbasell) comprenant des particules sphériques d'oxyde de zinc de diamètre moyen 0,50 µm, de surface spécifique d'environ 15 m²/g, synthétisées par le procédé décrit dans la demande de brevet déposée sous le numéro FR 14 54141. La quantité de particules sphériques d'oxyde de zinc est de 2 % en masse par rapport à la masse totale.

L'échantillon témoin est une plaquette de polyéthylène basse densité (Purell PE 1840 H commercialisé par la société Lyondelbasell).

Les souches de référence sont les suivantes : *Escherichia coli* CIP 53126 et *Staphylococcus aureus* CIP 53156.

Les plaquettes ont été traitées à l'alcool. L'ensemble des plaquettes a été rincé à l'eau distillée stérile, puis séchées sous hotte à flux laminaire avant l'essai. Le film de recouvrement est un film stérile de sacs Stomacher (40 mm x 40 mm) (AES).

Les suspensions ont été préparées avec la solution Nutrient Broth diluée au 1/500. La solution de récupération est la solution SCDLP préconisée par la norme. Les dilutions ultérieures sont réalisées en PBS (tampon phosphate salin). Le milieu est une gélose trypcase soja (Biomérieux). Les dépôts sont réalisés sur les plaquettes en une quantité de 1,83.10⁵ UFC (Unités Formant une Colonie). La prolifération est mesurée 24 heures après le dépôt, ou 1h après le dépôt (pour *Streptococcus pneumoniae et Haemophilus influenzae).*

Les résultats sont présentés dans le tableau 1 ci-dessous, la valeur de contamination étant ramenée à 1 pour la plaquette témoin (sans particules).

**Tableau 1**

| Souche | Prolifération pour la plaquette selon l'invention | Prolifération pour la plaquette témoin |
|---|---|---|
| *Escherichia coli* | 0,000628765 | 1 |
| *Staphylococcus aureus* | 0,009632989 | 1 |
| *Pseudomonas aeruginosa* | 0,459916667 | 1 |
| *Listeria monocytogenes* | 6,21.10⁻⁵ | 1 |
| *Salmonella enterica* | 1,43.10⁻⁶ | 1 |
| *Staphylococcus epidermidis* | 0,011402338 | 1 |
| *Streptococcus pneumoniae* | 0,41005694 | 1 |
| *Haemophilus influenzae* | 0,300787534 | 1 |

La prolifération sur les plaquettes formées avec un matériau selon l'invention est nettement plus faible que pour des plaquettes ne comprenant pas de particules, quel que soit le type de bactéries considéré.

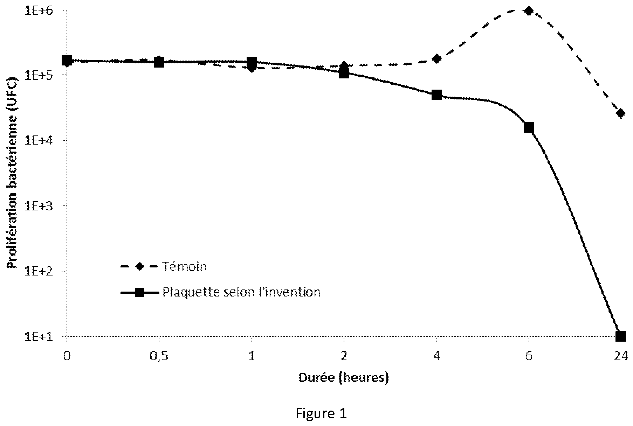

La prolifération des bactéries a été suivie tout au long des 24 heures d'essai pour *Escherichia coli.* La figure 1 présente la cinétique de diminution de la prolifération pour les plaquettes du matériau selon l'invention et pour la plaquette témoin.

D'autres plaquettes de polyéthylène basse densité (Purell PE 1840 H commercialisé par la société Lyondelbasell) comprenant des particules sphériques telles que spécifiées ci-dessus peuvent être préparées selon des quantités de particules variant de 0,2 à 2,5% en masse par rapport à la masse totale.

### Exemple 2 : Etude de l'influence du matériau selon l'invention sur la quantité de conservateurs

De la solution nutritive (Nutrient Broth) est déposée sur une plaquette de matériau selon l'invention et sur une plaquette témoin, telles que définies à l'exemple 1. On a fait varier la dose de conservateur (méthylparabène) dans la solution Nutrient Broth et le taux de prolifération d'*Escherichia coli* a été comparé dans les différentes conditions après 6 et 24 heures d'essai. Les quantités de conservateur sont exprimées en pourcentage en masse de la solution.

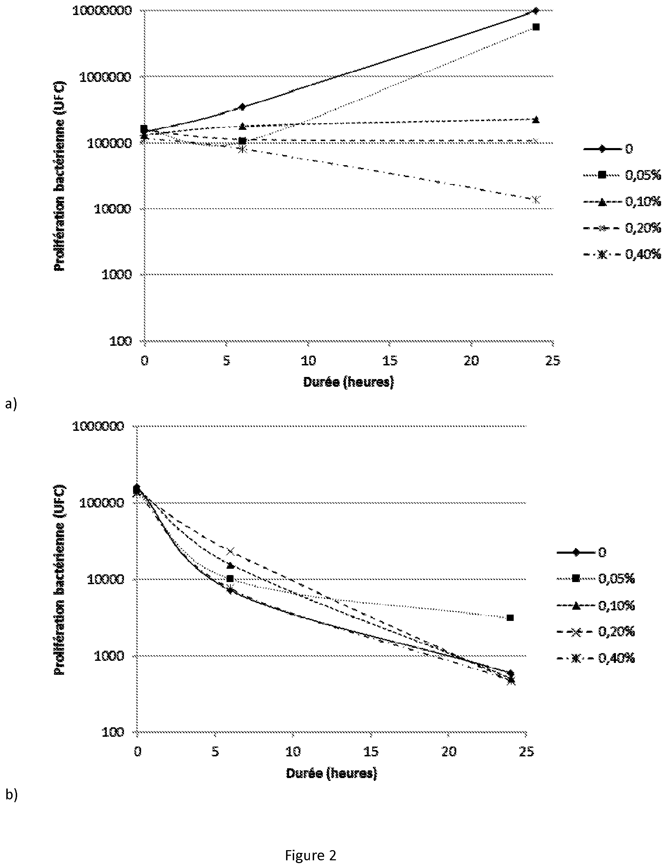

La figure 2 présente l'évolution de la prolifération des bactéries en fonction du taux de conservateur pour la plaquette témoin (a) et pour la plaquette en matériau selon l'invention (b).

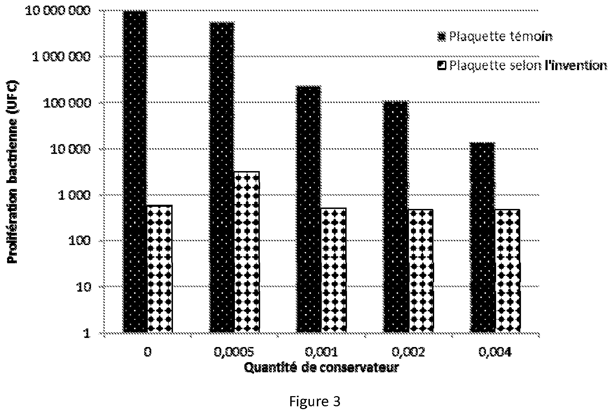

La figure 3 présente la comparaison des activités antibactériennes de la plaquette témoin et de la plaquette en matériau selon l'invention en fonction de la quantité de conservateur.

On voit qu'une activité antibactérienne aussi faible peut être obtenue pour la plaquette en matériau selon l'invention lorsque la solution contient seulement 0,1% de conservateur que pour une plaquette témoin quand la solution contient 0,4% de conservateur.

L'utilisation du matériau selon l'invention peut donc permettre de réduire la quantité de conservateur d'un facteur 4 dans une composition qui est mise en contact avec ledit matériau par rapport à une composition mise en contact avec un matériau ne contenant pas de particules.

### Exemple 3 : Etude de rétro-contamination

Cet exemple concerne l'évaluation du traitement d'un flacon pour contenance d'un produit type collyre, après simulation d'utilisation pendant 14 jours et application régulière de contaminants potentiels d'origine humaine (*Staphylococcus aureus*, *Pseudomonas aeruginosa* et *Bacillus subtilis*).

### Matériels et méthodes

Les souches suivantes sont utilisées : *Staphylococcus aureus* CIP 4.83, *Pseudomonas aeruginosa* CIP 82118 et *Bacillus subtilis* (forme sporulée) CIP 52.62. La conservation et l'entretien des souches sont effectués selon la norme EN 12353 (Septembre 2006). Les suspensions bactériennes mères sont réalisées conformément à la pharmacopée européenne (7^{ème} édition, 2012, Chap. 2.6.12). Les suspensions de travail sont préparées à partir des supensions mères en réalisant six dilutions successives au dixième dans un liquide de suspension stérile contenant 9 g/L de tryptone-sel (correspondant à un ajustement théorique à 1.10² à 3.10² UFC/mL). Les suspensions sont dénombrées par ensemencement sur plaques. Le flacon de référence comprend un flacon, un embout et un bouchon en polyéthylène (Purell 1840H). Le flacon de l'essai comprend un flacon, un embout et un bouchon en matériau selon l'invention (polyéthylène Purell 1840H contenant des particules sphériques de ZnO de diamètre moyen 500 nm et de surface spécifique d'environ 15 m²/g). Le flacon, l'embout et le bouchon comprennent 2% en masse de particules sphériques de ZnO par rapport à la masse totale.

Les deux flacons sont remplis avec 9 mL de bouillon Nutrient Broth (3g d'extrait de viande, 10g de peptone de soja, 5g de NaCl, qsp 1L) au 1/500. Le pH est compris entre 6,8 et 7,2. Le protocole est appliqué sur 10 flacons d'essai et 10 flacons témoins.

Pendant 2 semaines, 8 simulations de prise sont réalisées, à raison de 4 simulations par semaine sur les deux types de flacons. Le mode opératoire de simulation de prise est reproduit à deux reprises dans la journée.

Le mode opératoire de simulation de prise est le suivant :
- Préparation des suspensions de travail des trois microorganismes comme décrit ci-avant,
- Préparation d'un mélange extemporané de 10 mL comprenant les trois suspensions à parts égales,
- Imbibage *ad libitum* d'un écouvillon stérile avec cette suspension,
- Dévissage du bouchon, prélèvement d'une goutte de produit par la voie normale d'utilisation,
- Simulation de l'utilisation par application de l'écouvillon imbibé sur la zone de distribution du produit, et
- Revissage du bouchon et placement des flacons à température ambiante jusqu'à la simulation suivante.

A 7 et 14 jours, la concentration de microorganismes présents dans le bouillon est évaluée. A 14 jours, l'état stérile du bouillon est également vérifié pour le flacon de l'essai.

Pour le dénombrement, l'essai a été réalisé selon les préconisations du chapitre 2.6.12 de la Pharmacopée Européenne. Après homogénéisation du contenu du flacon par passage au vortex, 2 x 100µL ont été introduits dans du milieu gélosé Trycase-soj a (Biomérieux) pour la flore aérobie totale et de Sabouraud (AES) pour la flore fongique totale. Les boites ont été incubées à 32,5 ± 2,5°C pendant 3 à 5 jours pour le milieu Trypcase-soja et 22,5 ± 2,5°C pendant 5 à 7 jours pour le milieu de Sabouraud.

Pour l'évaluation de la stérilité, l'essai a été réalisé selon les préconisations du chapitre 2.6.1 de la Pharmacopée Européenne. Il n'a été réalisé que sur les flacons en matériau selon l'invention et selon deux modes de prélèvements.

Pour les flacons N°1 à N°5, le bouillon a été collecté après passage à travers l'embout, pour se positionner dans des conditions les plus défavorables.

Pour les flacons N°6 à N°10, le bouillon a été collecté directement à l'intérieur du flacon après avoir dévissé et ôté l'embout.

La totalité du bouillon (environ 8,5mL) contenu dans les différents flacons a été prélevée puis introduite dans 100mL de bouillon Trycase-soja (Biomérieux). L'incubation a été réalisée à 22,5 ± 2,5°C pendant 14 jours. L'observation d'un trouble met en évidence la positivité de l'essai. Dans ce cas-là seulement, il a été procédé à un isolement afin de permettre une identification de la contamination.

Deux types de témoins ont été réalisés :

### Témoin négatif :

Un flacon en polyéthylène contenant 9 mL de bouillon a été placé dans les mêmes conditions d'ambiance que les flacons soumis à l'essai pour valider l'état stérile après 14 jours de simulation.

### Témoin positif :

Un flacon en polyéthylène contenant 9 mL de bouillon a été inoculé avec les trois bactéries (concentration finale d'environ 3 UFC/mL) et placé dans les mêmes conditions d'ambiance que les flacons soumis à l'essai pour valider le maintien de la viabilité des microorganismes durant les 14 jours de simulation.

### Résultats

Les résultats présentés dans les tableaux ci-dessous sont exprimés en nombre d'UFC pour 100 microlitres de bouillon.

### ➢ Dénombrement

Le tableau 2 ci-dessous présente les résultats de dénombrement à 7 et 14 jours sur milieu gélosé Trycase-soja. Les flacons selon l'invention sont les flacons « PE + ZnO », les autres flacons (« Flacons PE ») sont les flacons de référence en polyéthylène.

**Tableau 2**

| | | **7 jours** | | | **14 jours** | |
|---|---|---|---|---|---|---|
| **Echantillon** | **Flacon PE** | | **Flacon PE + ZnO** | **Flacon PE** | | **Flacon PE + ZnO** |
| **N°1** | 33** | | < 1 | > 300** | | < 1 |
| **N°3** | 2** | | < 1 | > 300** | | < 1 |
| **N°4** | 6* | | < 1 | > 300** | | < 1 |
| **N°5** | 1** | | < 1 | > 300** | | 2** |
| **N°6** | > 300* | | < 1 | > 300** | | < 1 |
| **N°7** | 1** | | < 1 | > 300** | | < 1 |
| **N°8** | 1** | | < 1 | > 300** | | < 1 |
| **N°9** | > 300** | | < 1 | > 300** | | < 1 |
| **N°10** | > 300** | | < 1 | > 300** | | < 1 |
| **Témoin Négatif** | < 1 | | < 1 | < 1 UFC | | < 1 |
| **Témoin positif** | > 300* | | > 300* | > 300* | | > 300* |

| | | | | | | |
|---|---|---|---|---|---|---|
| * Détection de *Pseudomonas aeruginosa* et *Bacillus subtilis* ** Détection majoritaire de *Pseudomonas aeruginosa* | | | | | | |

Le tableau 3 ci-dessous présente les résultats de dénombrement à 7 et 14 jours sur milieu de Sabouraud. Les flacons selon l'invention sont les flacons « PE + ZnO », les autres flacons (« Flacons PE ») sont les flacons de référence en polyéthylène.

**Tableau 3**

| | | **T7 jours** | | | **T14 jours** | |
|---|---|---|---|---|---|---|
| **Echantillon** | **Flacon PE** | | **Flacon PE + ZnO** | **Flacon PE** | | **Flacon PE + ZnO** |
| **N°1** | 28 | | < 1 | > 300 | | < 1 |
| **N°2** | 5 | | < 1 | > 300 | | < 1 |
| **N°3** | 2 | | < 1 | > 300 | | < 1 |
| **N°4** | 13 | | < 1 | > 300 | | < 1 |
| **N°5** | 1 | | < 1 | > 300 | | 1 |
| **N°6** | > 300 | | < 1 | > 300 | | < 1 |
| **N°7** | 1 | | < 1 | > 300 | | < 1 |
| **N°8** | < 1 | | < 1 | > 300 | | < 1 |
| **N°9** | > 300 | | < 1 | > 300 | | < 1 |
| **N°10** | > 300 | | < 1 | > 300 | | < 1 |
| **Témoin Négatif** | < 1 | | < 1 | < 1 | | < 1 |
| **Témoin positif** | > 300 | | > 300 | > 300 | | > 300 |

La population observée sur gélose de Sabouraud est principalement d'origine bactérienne (majoritairement *Pseudomonas aeruginosa*) sauf pour le flacon N°6 en polyéthylène où la présence de moisissures a été détectée en plus d'une flore bactérienne majoritaire.

Après 7 jours de simulation, aucun flacon sur les 10 soumis à l'essai ne met en évidence la présence de contamination pour la série PE + ZnO (flacons selon l'invention). Dans le même temps, 9 flacons de la série PE (flacons de référence) sur les 10 soumis à l'essai sont contaminés, le niveau de contamination étant hétérogène de 1 UFC détecté à > 300UFC.

Après 14 jours de simulation, un seul flacon sur les 10 soumis à l'essai pour la série PE + ZnO (flacons selon l'invention) met en évidence la présence de *Pseudomonas aeruginosa.* Dans le même temps, tous les flacons de la série PE (flacons de référence) sont contaminés avec un niveau de contamination important puisque supérieur à 300UFC pour 100µL de bouillon.

### ➢ Evaluation de la stérilité

Le tableau 4 ci-dessous rassemble les résultats de contrôle de la stérilité pour les flacons selon l'invention dans lesquels le prélèvement a été réalisé directement à l'intérieur du flacon.

**Tableau 4**

| **Echantillon** | **Croissance** | **Identification** |
|---|---|---|
| **N°6** | Négative | - |
| **N°7** | Négative | - |
| **N°8** | Négative | - |
| **N°9** | Négative | - |
| **N°10** | Négative | - |
| **Témoin Négatif** | Négative | - |
| **Témoin positif** | Positive | *Pseudomonas aeruginosa Staphylococcus aureus* |

Les résultats démontrent une préservation de l'état stérile du bouillon pour les 5 flacons où le prélèvement a été directement réalisé à l'intérieur du contenant.

### ➢ Conclusion

L'utilisation d'un matériau selon l'invention au niveau du flacon et de l'embout permet de limiter le risque de contamination du bouillon dans les conditions testées, à savoir contamination artificielle au niveau de l'embout, associant une bactérie à Gram + (*S*. *aureus*), une bactérie à Gram - (*P. aeruginosa*) et une bactérie à Gram - sous forme sporulée (*Bacillus subtilis*).

Dans les mêmes conditions, l'utilisation de flacons de polyéthylène est caractérisée par une rétro-contamination avec croissance significative d'au moins 1 des 3 contaminants présents. Les contrôles de stérilité réalisés sur les flacons selon l'invention confirment l'absence de rétro-contamination du produit contenu dans le conditionnement.

### Exemple 4 : Etude d'activité antimicrobienne

L'ensemble des matériaux exemplifiés ci-dessus (inorganique et organique, comme les plastique, caoutchouc, vernis, peinture, textile, silicone, colle, revêtements, élastomère) a été testé selon la norme ISO22196 et les résultats obtenus correspondent à une activité antibactérienne sur *Escherichia coli* des échantillons comprise entre 1 et 7 UFC/cm². Le taux de particules en masse par rapport à la masse totale (masse de la matrice et de particules) est de 0,2 à 2 % et les surfaces spécifiques des particules sont de 15 à 30 m²/g pour un diamètre moyen de particules de 0,50 µm.

Le test décrit par la norme nécessite l'utilisation de 3 échantillons traités (40mm x 40mm) et 6 échantillons non traités pour chaque microorganisme à analyser.
A. Inoculum de concentration connue du microorganisme à tester déposé de façon homogène sur la surface des échantillons,
B. Détermination de la concentration en microorganismes viables réalisée immédiatement après inoculation et suite à l'incubation 24 heures par la méthode de culture sur milieu gélosé,
C. La comparaison de ces numérations permet de déterminer la valeur de l'activité antimicrobienne de la surface analysée.

Les supports sont traités à l'alcool. L'ensemble des supports est rincé à l'eau distillée stérile, puis séché sous hotte à flux laminaire avant essai.

Film stérile de sacs Stomacher (40mm x 40mm).

Conditions d'essais :
- Température de contact : 36 ± 1°C
- Humidité relative : 80 %
- temps de contact : 24 heures

### Résultats

Pour des particules de MgO :
MgO avec une matrice de polyéthylène de basse densité de RIBLENE (essais selon ISO22196 *E. coli)*
T0 témoin : 1,38.10⁵ UFC
T0 échantillon : 1,44.10⁵ UFC
T24h témoin : 2,83.10⁷ UFC
T24h échantillon : 60 UFC

Pour des particules de ZnO et de MgO (quantités : 0,5 % et de 0.2% en masse respectivement par rapport à la masse totale) :
ZnO/MgO (ratio pondéral 2.5) avec une matrice de polyéthylène de basse densité Purell PE 1840 H (essais selon ISO22196 *E. coli*)
T0 témoin : 1,67.10⁵ UFC
T0 échantillon : 1,72.10⁵ UFC
T24h témoin : 2,56.10⁷ UFC
T24h échantillon : 196 UFC

## Revendications

1. Utilisation d'un matériau solide comprenant une matrice et un ensemble de microparticules comprenant ou constituées d'au moins un agent antimicrobien pour prévenir, limiter et/ou supprimer la contamination dudit matériau et/ou la contamination d'une composition qui est en contact avec ledit matériau pendant au moins un temps donné, et/ou prévenir, supprimer et/ou ralentir la formation de biofilms à la surface dudit matériau, dans laquelle l'agent antimicrobien ne migre pas à l'extérieur dudit matériau, et dans laquelle lesdites microparticules comprennent de l'oxyde de zinc ou sont constituées d'oxyde de zinc (ZnO) ou comprennent de l'oxyde de magnésium ou sont constituées d'oxyde de magnésium (MgO), ou un mélange d'oxyde de magnésium et d'oxyde de zinc,
dans laquelle la quantité de microparticules est de 0,1 à 10% en masse par rapport à la masse de la matrice et de particules,
dans laquelle les microparticules ont un diamètre moyen compris entre 0,1 et 5 micromètres, déterminé par microscopie, et
dans laquelle la matrice est une matrice organique, **caractérisé en ce que** lesdites microparticules sont sphériques et présentent un coefficient de sphéricité supérieur ou égal à 0,9 ,
le coefficient de sphéricité d'une particule étant le rapport du plus petit diamètre de la particule au plus grand diamètre de celle-ci, déterminé par microscopie.

2. Utilisation selon la revendication 1, dans laquelle la quantité de microparticules est de 0,1 à 5%, plus spécifiquement de 0,5 à 3%, ou de 1 à 3%, en masse par rapport à la masse de la matrice et de particules.

3. Utilisation selon la revendication 1 ou 2, dans laquelle les microparticules ont un diamètre moyen compris entre 0,4 et 5 micromètres.

4. Utilisation selon l'une des revendications précédentes, dans laquelle au moins 50%, au moins 60%, au moins 70%, au moins 80%, au moins 90%, au moins 95% en nombre desdites microparticules de l'ensemble ont une sphéricité telle que définie dans la revendication 1.

5. Utilisation selon l'une des revendications précédentes, dans laquelle les microparticules présentent des surfaces spécifiques supérieures ou égales à 15 m²/g.

6. Utilisation selon l'une des revendications précédentes, dans laquelle les microparticules sont choisies parmi des microparticules de ZnO, des microparticules de ZnO dopé au sodium ou à l'aluminium, et des microparticules mésostructurées comprenant du ZnO.

7. Utilisation selon l'une quelconque des revendications 1 à 6, dans laquelle la matrice est une matrice polymérique, de préférence la matrice polymérique est une matrice polymérique thermoplastique choisie parmi le copolymère acrylonitrile butadiène styrène, l'acétate de cellulose, le polystyrène, notamment le polystyrène expansé, les polyamides, le poly(téréphtalate de butylène), les polycarbonates, le polyéthylène, le poly(téréphtalate d'éthylène), le poly(méthacrylate de méthyle), le polyformaldéhyde, le polypropylène, le poly(acétate de vinyle), le poly(chlorure de vinyle), le poly(acide lactique) (PLA), la polycaprolactone, le polyhydroxyalcanoate (PHA), les polysaccharides et le copolymère styrène-acrylonitrᵢₗₑ.

8. Utilisation selon l'une quelconque des revendications 1 à 7, dans laquelle la composition est physiologiquement acceptable pour un mammifère.

9. Utilisation selon l'une des revendications 1 à 8, pour fabriquer un article susceptible d'être mis en contact avec au moins une source de contamination microbienne, dans laquelle l'article est tout ou partie d'un emballage, contenant ou dispositif de distribution d'une composition alimentaire, diététique, cosmétique, dermatologique ou pharmaceutique.

10. Article constitué d'un matériau solide comprenant une matrice et un ensemble de microparticules comprenant ou constituées d'au moins un agent antimicrobien, ledit matériau étant tel que défini dans l'une des revendications 1 à 8.

11. Article selon la revendication 10, dans lequel l'article est tout ou partie d'un emballage, contenant ou dispositif de distribution d'une composition alimentaire, diététique, cosmétique, dermatologique ou pharmaceutique.

12. Article selon l'une des revendications 10 et 11, dans lequel l'article est choisi parmi des bouchons, des opercules, des joints, des capsules, des couvercles, des bondes et des robinets destinés à la fermeture de bouteilles, de flacons, de pots, de boîtes, de bidons, de barriques, de cuves, ou de divers récipients utilisés pour le conditionnement et/ou le stockage de produits alimentaires, diététiques, cosmétiques, dermatologiques ou pharmaceutiques.

13. Article selon l'une des revendications 10 et 11, dans lequel l'article est tout ou partie de bouteilles, de flacons, de pots, de boîtes, de bidons, de barriques, de cuves ou de divers récipients utilisés pour le conditionnement et/ou le stockage de produits alimentaires, diététiques, cosmétiques, dermatologiques ou pharmaceutiques.

14. Procédé de fabrication d'un article, dans lequel ledit procédé comprend une étape de mise forme d'un matériau solide comprenant une matrice et un ensemble de microparticules comprenant ou constituées d'au moins un agent antimicrobien, ledit matériau solide étant tel que défini dans l'une des revendications 1 à 8 et comprend une étape de mise en forme dudit article.

## Patentansprüche

1. Verwendung eines festen Materials, umfassend eine Matrix und eine Reihe an Mikropartikeln, umfassend oder gebildet aus wenigstens einem antimikrobiellen Agens, um die Kontamination des Materials und/oder die Kontamination einer Zusammensetzung, die in Kontakt mit dem Material über wenigstens eine gegebene Zeit ist, zu verhindern, zu begrenzen und/oder zu supprimieren und/oder die Bildung von Biofilmen auf der Oberfläche des Materials zu verhindern, zu supprimieren und/oder zu verzögern, wobei das antimikrobielle Agens nicht auf die Außenseite des Materials migriert, und wobei die Mikropartikel Zinkoxid umfassen oder aus Zinkoxid (ZnO) gebildet sind oder Magnesiumoxid umfassen oder aus Magnesiumoxid (MgO) oder aus einer Mischung aus Magnesiumoxid und Zinkoxid gebildet sind,
wobei die Menge an Mikropartikeln 0,1 bis 10 Gew.-% bezogen auf das Gewicht der Matrix und Partikel beträgt,
wobei die Mikropartikel einen mikroskopisch bestimmten mittleren Durchmesser zwischen 0,1 und 5 Mikrometer besitzen, und
wobei die Matrix eine organische Matrix ist, **dadurch gekennzeichnet, dass** die Mikropartikel kugelförmig sind und einen Kugel-Koeffizienten größer oder gleich 0,9 aufweisen,
wobei der Kugel-Koeffizient eines Partikels das Verhältnis des mikroskopisch bestimmten kleinsten Durchmessers des Mikropartikels zu dessen größtem Durchmesser ist.

2. Verwendung gemäß Anspruch 1, wobei die Menge an Mikropartikeln 0,1 bis 5%, spezifischer 0,5 bis 3 oder 1 bis 3 Gew.-% bezogen auf das Gewicht der Matrix und Partikel beträgt.

3. Verwendung gemäß Anspruch 1 oder 2, wobei die Mikropartikel einen mittleren Durchmesser zwischen 0,4 und 5 Mikrometer besitzen.

4. Verwendung gemäß einem der vorhergehenden Ansprüche, wobei zahlenmäßig wenigstens 50%, wenigstens 60%, wenigstens 70%, wenigstens 80%, wenigstens 90%, wenigstens 95% der gesamten Mikropartikel kugelförmig sind, wie in Anspruch 1 definiert.

5. Verwendung gemäß einem der vorhergehenden Ansprüche, wobei die Mikropartikel spezifische Oberflächen größer oder gleich 15 m²/g aufweisen.

6. Verwendung gemäß einem der vorhergehenden Ansprüche, wobei die Mikropartikel aus ZnO-Mikropartikeln, mit Natrium oder Aluminium dotierten ZnO-Mikropartikeln, und ZnO umfassenden mesostrukturierten Mikropartikeln ausgewählt sind.

7. Verwendung gemäß einem der Ansprüche 1 bis 6, wobei die Matrix eine Polymermatrix ist, vorzugsweise die Polymermatrix eine thermoplastische Polymermatrix ist, ausgewählt aus Acrylnitril-Butadien-Styrol-Copolymer, Celluloseacetat, Polystyrol, insbesondere expandiertem Polystyrol, Polyamiden, Poly(butylenterephthalat), Polycarbonaten, Polyethylen, Poly(ethylenterephthalat), Poly(methylmethacrylat), Polyformaldehyd, Polypropylen, Poly(vinylacetat), Poly(vinylchlorid), Poly(milchsäure) (PLA), Polycaprolacton, Polyhydroxyalkanoat (PHA), Polysacchariden und Acrylnitril-Styrol-Copolymer.

8. Verwendung gemäß einem der Ansprüche 1 bis 7, wobei die Zusammensetzung für ein Säugetier physiologisch verträglich ist.

9. Verwendung gemäß einem der Ansprüche 1 bis 8, zur Herstellung eines Gegenstandes, der mit wenigstens einer mikrobiellen Kontaminationsquelle in Kontakt kommen kann, wobei der Gegenstand vollständig oder teilweise eine Verpackung, ein Behälter oder eine Vorrichtung zur Abgabe einer Nahrungsmittelzusammensetzung, einer diätetischen, kosmetischen, dermatologischen oder pharmazeutischen Zusammensetzung ist.

10. Gegenstand, gebildet aus einem festen Material, umfassend eine Matrix und eine Reihe an Mikropartikeln, umfassend oder gebildet aus wenigstens einem antimikrobiellen Agens, wobei das Material wie in einem der Ansprüche 1 bis 8 definiert ist.

11. Gegenstand gemäß Anspruch 10, wobei der Gegenstand ganz oder teilweise eine Verpackung, ein Behälter oder eine Vorrichtung zur Abgabe einer Nahrungsmittelzusammensetzung, einer diätetischen, kosmetischen, dermatologischen oder pharmazeutischen Zusammensetzung ist.

12. Gegenstand gemäß einem der Ansprüche 10 und 11, wobei der Gegenstand aus Stopfen, Verschlüssen, Dichtungen, Kapseln, Kappen, Stöpseln und Sperrhähnen ausgewählt ist, die zum Verschließen von Flaschen, Kolben, Tiegeln, Dosen, Kanistern, Fässern, Tanks oder verschiedenen Behältnissen geeignet sind, die zum Verpacken und/oder zur Lagerung von Nahrungsmitteln, diätetischen, kosmetischen, dermatologischen oder pharmazeutischen Produkten verwendet werden.

13. Gegenstand gemäß einem der Ansprüche 10 und 11, wobei der Gegenstand ganz oder teilweise Flaschen, Kolben, Tiegel, Dosen, Kanister, Fässer, Tanks oder verschiedene Behältnisse darstellt, die zum Verpacken und/oder zur Lagerung von Nahrungsmitteln, diätetischen, kosmetischen, dermatologischen oder pharmazeutischen Produkten verwendet werden.

14. Verfahren zur Herstellung eines Gegenstandes, wobei das Verfahren einen Schritt des Formens eines festen Materials umfasst, umfassend eine Matrix und eine Reihe an Mikropartikeln, umfassend oder gebildet aus wenigstens einem antimikrobiellen Agens, wobei das feste Material wie in einem der Ansprüche 1 bis 8 definiert ist und einen Schritt des Formens des Gegenstandes umfasst.

## Claims

1. The use of a solid material comprising a matrix and a set of microparticles comprising or consisting of at least one antimicrobial agent for preventing, limiting and/or suppressing the contamination of said material and/or the contamination of a composition which is in contact with the said material for at least a given time, and/or preventing, suppressing and/or slowing down biofilm formation on the surface of the said material, wherein the antimicrobial agent does not migrate outside of said material, and wherein said microparticles comprise zinc oxide or consist of zinc oxide (ZnO) or comprise magnesium oxide or consist of magnesium oxide (MgO), or a mixture of magnesium oxide and zinc oxide, wherein the quantity of microparticles is from 0.1 to 10% by weight relative to the weight of the matrix and particles,
wherein the microparticles have a mean diameter between 0.1 and 5 micrometres, determined by microscopy, and
wherein the matrix is an organic matrix, **characterized in that** said microparticles are spherical and have a coefficient of sphericity greater than or equal to 0.9,
the coefficient of sphericity of a particle being the ratio of the smallest diameter of the particle to the largest diameter thereof, determined by microscopy.

2. The use according to claim 1, wherein the quantity of microparticles is 0.1 to 5%, more specifically 0.5 to 3%, or 1 to 3%, by weight based on the weight of the matrix and particles.

3. The use according to claim 1 or 2, wherein the microparticles have an average diameter of between 0.4 and 5 micrometres.

4. The use according to one of the preceding claims, wherein at least 50%, at least 60%, at least 70%, at least 80%, at least 90%, at least 95% by number of said microparticles of the set have a sphericity as defined in claim 1.

5. The use according to one of the preceding claims, wherein the microparticles have specific surface areas greater than or equal to 15 m²/g.

6. The use according to one of the preceding claims, wherein the microparticles are chosen from ZnO microparticles, ZnO microparticles doped with sodium or aluminium, and mesostructured microparticles comprising ZnO.

7. The use according to any one of claims 1 to 6, wherein the matrix is a polymeric matrix, preferably the polymeric matrix is a thermoplastic polymeric matrix selected from acrylonitrile butadiene styrene copolymer, cellulose acetate, polystyrene, especially expanded polystyrene, polyamides, poly(butylene terephthalate), polycarbonates, polyethylene, poly(ethylene terephthalate), poly(methyl methacrylate), polyformaldehyde, polypropylene, poly(vinyl acetate), poly(vinyl chloride), poly(lactic acid) (PLA), polycaprolactone, polyhydroxyalkanoate (PHA), polysaccharides and the copolymer styrene-acrylonitrile.

8. The use according to any one of claims 1 to 7, wherein the composition is physiologically acceptable to a mammal.

9. The use according to one of claims 1 to 8, for manufacturing an article that may be brought into contact with at least one source of microbial contamination, wherein the article is all or part of a package, container or delivery device for a food, dietary, cosmetic, dermatological or pharmaceutical composition.

10. The article consisting of a solid material comprising a matrix and a set of microparticles comprising or consisting of at least one antimicrobial agent, said material being as defined in one of claims 1 to 8.

11. The article according to claim 10, wherein the article is all or part of a package, container or delivery device for a food, dietary, cosmetic, dermatological or pharmaceutical composition.

12. The article according to one of claims 10 and 11, wherein the article is chosen from stoppers, covers, seals, capsules, lids, plugs and taps intended for the closure of bottles, flasks, jars, boxes, cans, barrels, tanks, or various containers used for the packaging and/or storage of food, dietary, cosmetic, dermatological or pharmaceutical products.

13. The article according to one of claims 10 and 11, wherein the article forms all or part of bottles, flasks, jars, boxes, cans, barrels, tanks or various containers used for packaging and /or storage of food, dietary, cosmetic, dermatological or pharmaceutical products.

14. The method of manufacturing an article, wherein said method comprises a step of forming a solid material comprising a matrix and a set of microparticles comprising or consisting of at least one antimicrobial agent, said solid material being as defined in one of claims 1 to 8 and comprises a step of forming said article.
